# EUROPEAN PATENT SPECIFICATION

(11) **EP 4 299 574 B1**
(45) Date of publication and mention of the grant of the patent: **02.04.2025**
(21) Application number: 22893209.1
(22) Date of filing: 10.11.2022
(51) Int. Cl.: C07D 403/10, H10K 50/00, C09K 11/06

(54) **NOVEL COMPOUND AND ORGANIC LIGHT-EMITTING DEVICE COMPRISING SAME**
NEUARTIGE VERBINDUNG UND ORGANISCHE LICHTEMITTIERENDE VORRICHTUNG DAMIT
NOUVEAU COMPOSÉ ET DISPOSITIF ÉLECTROLUMINESCENT ORGANIQUE LE COMPRENANT

(30) Priority: 10.11.2021 KR 20210153679; 09.11.2022 KR 20220148749
(43) Date of publication of application: 03.01.2024
(73) Proprietor: LG Chem, Ltd., Seoul 07336 (KR)
(72) Inventor: LEE, Da Jung, Daejeon 34122 (KR); LEE, Dong Hoon, Daejeon 34122 (KR); KIM, Minjun, Daejeon 34122 (KR); SUH, Sang Duk, Daejeon 34122 (KR); KIM, Young Seok, Daejeon 34122 (KR); KIM, Donghee, Daejeon 34122 (KR); OH, Joongsuk, Daejeon 34122 (KR)
(74) Representative: Hoffmann Eitle
(86) International application number: PCT/KR2022/017604
(87) International publication number: WO 2023/085790

(56) References cited:
- EP-A1- 3 675 194
- JP-A- 2019 172 608
- JP-A- 2019 199 442
- KR-A- 20190 086 347
- KR-A- 20190 140 233
- KR-A- 20200 083 171
- KR-A- 20220 000 856
- US-A1- 2019 214 570
- US-B1- 11 063 226

## Description

### [TECHNICAL FIELD]

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuous need to develop a new material for the organic material used in the organic light emitting device as described above.

Patent Literature documents 1 to 6 cited below describe organic light-emitting devices comprising: a first electrode; a second electrode; and an organic layer disposed between the first electrode and wherein the organic layer comprises an emission layer comprising a carbazole-containing heterocyclic compounds as described therein.

### [Prior Art Literature]

### [Patent Literature]

(Patent Literature 1) Korean Unexamined Patent Publication No. 10-2000-0051826
(Patent Literature 2) US 11 063 226 B1
(Patent Literature 3) US 2019/214570 A1
(Patent Literature 4) EP 3 675 194 A1
(Patent Literature 5) JP 2019 172608 A
(Patent Literature 6) JP 2019 199442 A

### [DETAILED DESCRIPTION OF THE INVENTION]

### [Technical Problem]

It is an object of the present disclosure to provide a novel compound and an organic light emitting device comprising the same.

### [Technical Solution]

According to one aspect of the present disclosure, there is provided a compound represented by the following Chemical Formula 1: 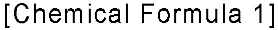 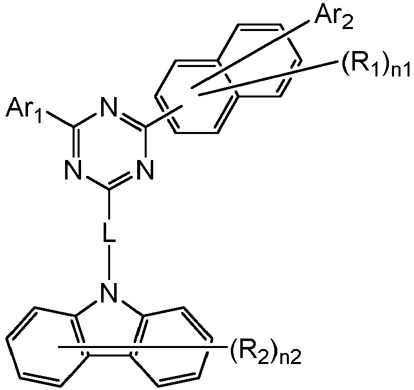 wherein, in Chemical Formula 1,
Ar₁, Ar₂ and L are as defined in the appended claims and discussed further below,
R₁ and R₂ are each independently hydrogen or deuterium,
n1 is an integer of 0 to 6, and
n2 is an integer of 0 to 8.

According to another aspect of the present disclosure, there is provided an organic light emitting device comprising: a first electrode; a second electrode that is provided opposite to the first electrode; and one or more organic material layers that are provided between the first electrode and the second electrode, wherein one or more layers of the organic material layers comprises the compound represented by Chemical Formula 1.

### [ADVANTAGEOUS EFFECTS]

The above-mentioned compound represented by Chemical Formula 1 can be used as a material of an organic material layer in an organic light emitting device, and can improve the efficiency, achieve low driving voltage and/or improve lifetime characteristics in the organic light emitting device. In particular, the compound represented by the Chemical Formula 1 can be used as a hole injection material, hole transport material, light emitting material, electron transport material, or electron injection material.

### [BRIEF DESCRIPTION OF THE DRAWINGS]

Fig. 1 shows an example of an organic light emitting device comprising a substrate 1, an anode 2, a light emitting layer 3, and a cathode 4.
Fig. 2 shows an example of an organic light emitting device comprising a substrate 1, an anode 2, a hole injection layer 5, a hole transport layer 6, a light emitting layer 3, an electron injection and transport layer 7, and a cathode 4.

### [DETAILED DESCRIPTION OF THE EMBODIMENTS]

Hereinafter, embodiments of the present disclosure will be described in more detail to facilitate understanding of the invention.

The present disclosure provides the compound represented by the Chemical Formula 1.

As used herein, the notation 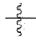 and 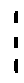 mean a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl group containing at least one of N, O and S atoms, or being unsubstituted or substituted with a substituent to which two or more substituents of the above-exemplified substituents are linked. For example, "a substituent in which two or more substituents are linked" may be a biphenyl group. Namely, a biphenyl group may be an aryl group, or it may also be interpreted as a substituent in which two phenyl groups are linked.

In the present disclosure, the carbon number of a carbonyl group is not particularly limited, but is preferably 1 to 40. Specifically, it may be a substituent having the following structure, but is not limited thereto.

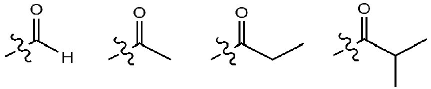

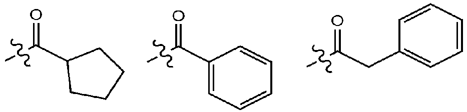

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, it may be a substituent having the following structure, but is not limited thereto.

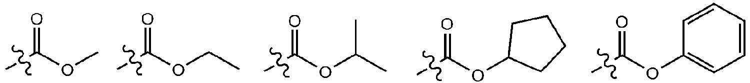

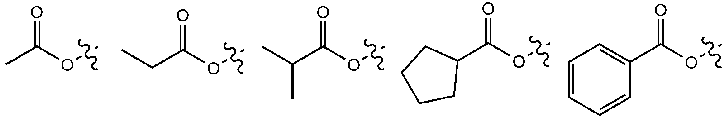

In the present disclosure, the carbon number of an imide group is not particularly limited, but is preferably 1 to 25. Specifically, it may be a substituent having the following structure, but is not limited thereto.

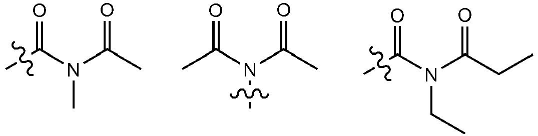

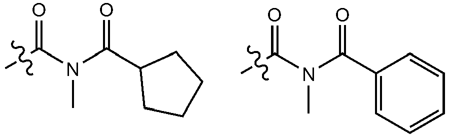

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, the boron group specifically includes a trimethylboron group, a triethyl boron group, a t-butyldimethylboron group, a triphenyl boron group, a phenyl boron group, and the like, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenylyl group, a terphenylyl group or the like as the monocyclic aryl group, but is not limited thereto. The polycyclic aryl group includes a naphthyl group, an anthracenyl group, a phenanthryl group, a pyrenyl group, a perylenyl group, a chrysenyl group, a fluorenyl group, or the like, but is not limited thereto.

In the present disclosure, the fluorenyl group may be substituted, and two substituents may be linked with each other to form a spiro structure. In the case where the fluorenyl group is substituted, 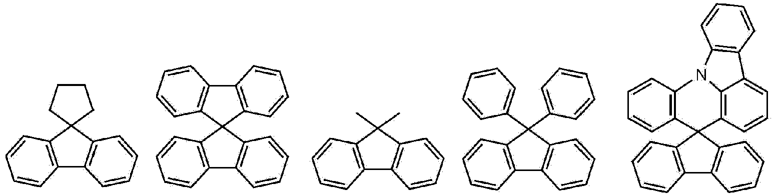 and the like can be formed.

In the present disclosure, a heteroaryl group is a heteroaryl group containing at least one of O, N, Si and S as a heteroatom, and the carbon number thereof is not particularly limited, but is preferably 2 to 60. According to one embodiment, the carbon number of the heteroaryl group is 6 to 30. According to one embodiment, the carbon number of the heteroaryl group is 6 to 20. Examples of the heteroaryl group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the above-mentioned examples of the aryl group. In the present disclosure, the alkyl group in the aralkyl group, the alkylaryl group and the alkylamine group is the same as the above-mentioned examples of the alkyl group. In the present disclosure, the heteroaryl in the heteroarylamine may be applied to the above-mentioned description of the heteroaryl group. In the present disclosure, the alkenyl group in the aralkenyl group is the same as the above-mentioned examples of the alkenyl group. In the present disclosure, the above-mentioned description of the aryl group may be applied except that the arylene is a divalent group. In the present disclosure, the above-mentioned description of the heteroaryl group can be applied except that the heteroarylene is a divalent group. In the present disclosure, the above-mentioned description of the aryl group or cycloalkyl group may be applied except that the hydrocarbon ring is not a monovalent group but formed by combining two substituent groups. In the present disclosure, the above-mentioned description of the heteroaryl group may be applied except that the heteroaryl is not a monovalent group but formed by combining two substituent groups.

Preferably, the Chemical Formula 1 may be represented by any one of the following Chemical Formulas 1-1 to 1-6: 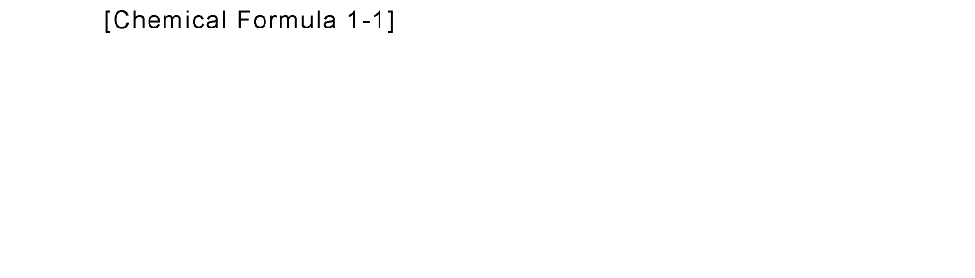 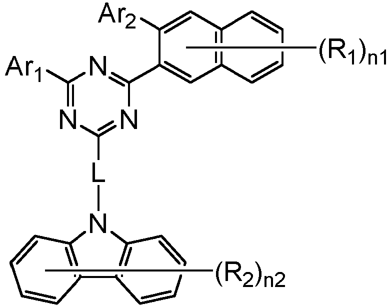 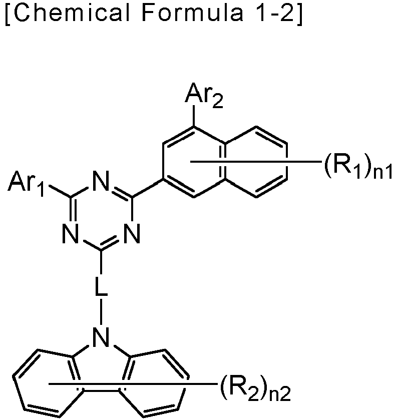 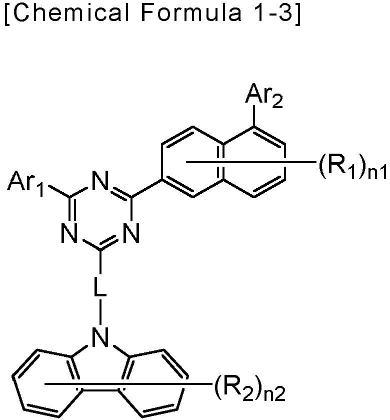 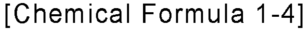 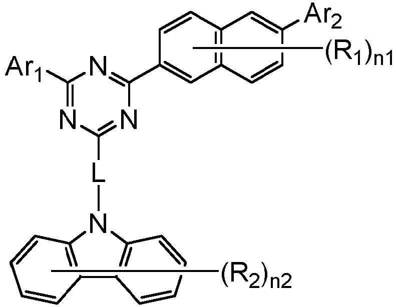 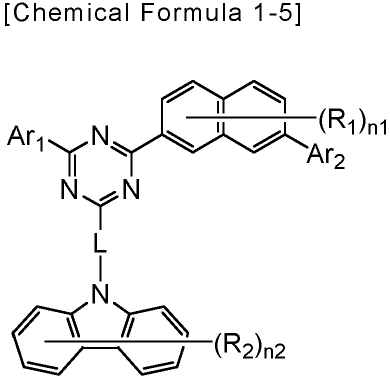 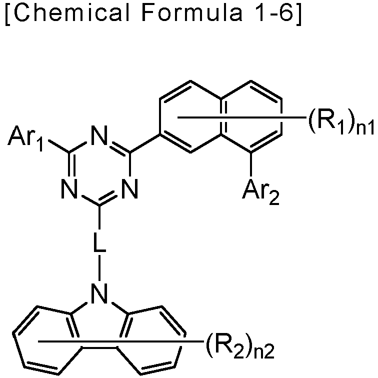 wherein, in Chemical Formulas 1-1 to 1-6,
Ar₁, Ar₂, L, R₁, R₂, n1 and n2 are as defined in Chemical Formula 1.

Ar₁ is any one selected from the group consisting of:

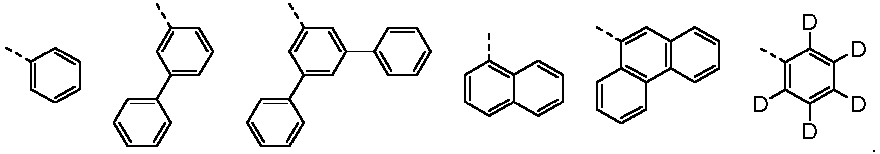

Ar₂ is phenyl or naphthyl, wherein the Ar₂ may be unsubstituted or substituted with at least one deuterium.

More preferably, Ar₂ may be selected from the group consisting of:

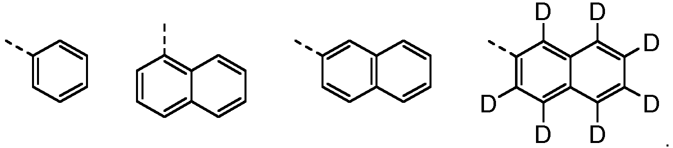

L is any one selected from the group consisting of:

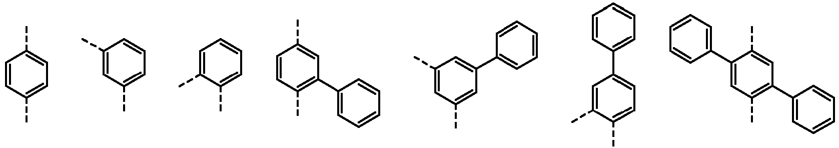

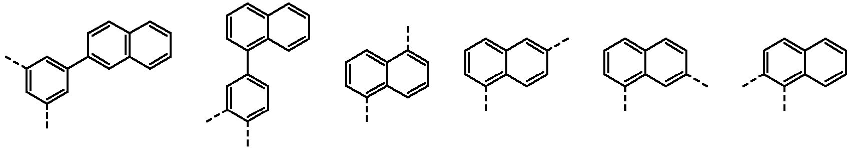

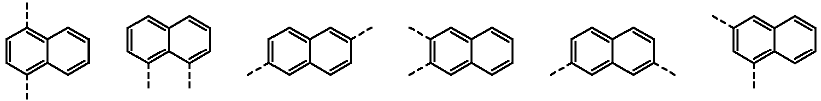

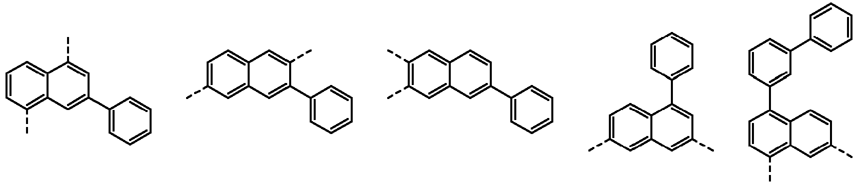

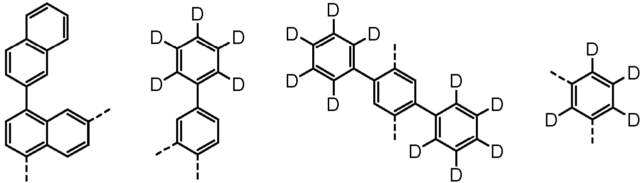

Preferably, R₁ and R₂ may be each independently hydrogen.

Representative examples of the compound represented by Chemical Formula 1 are as follows:

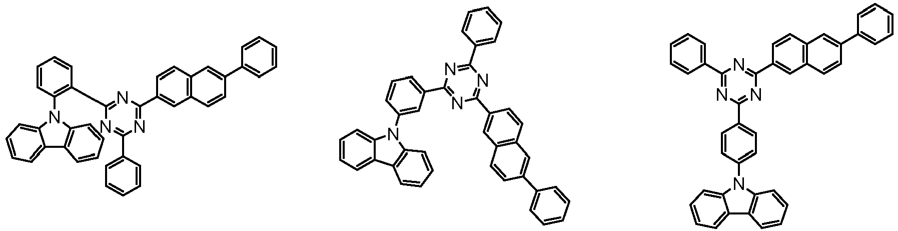

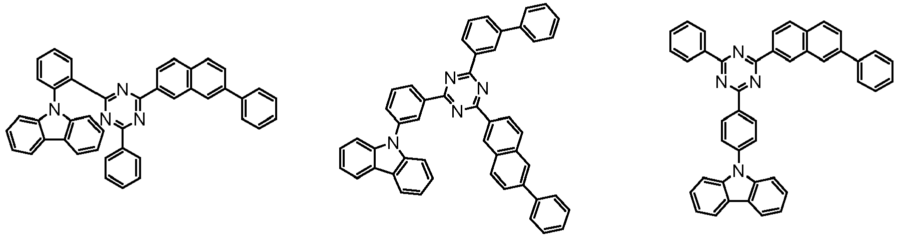

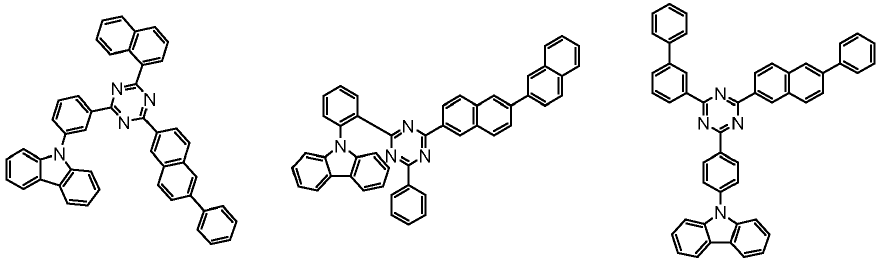

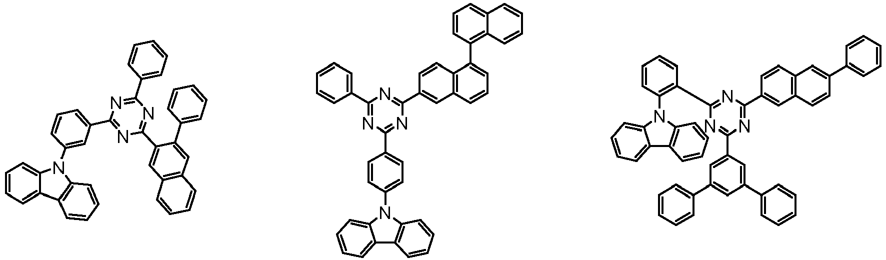

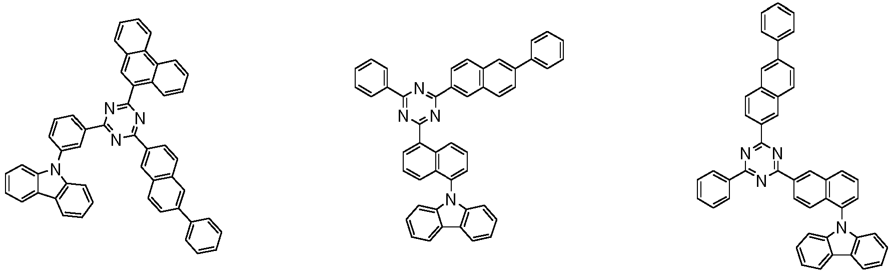

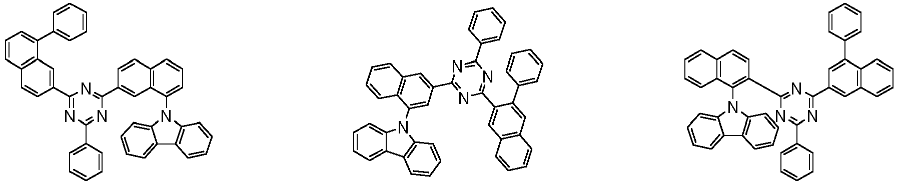

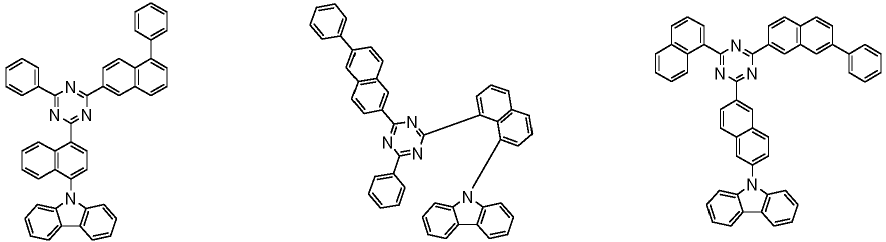

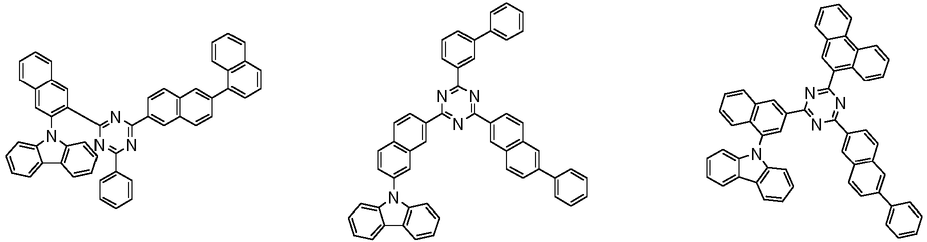

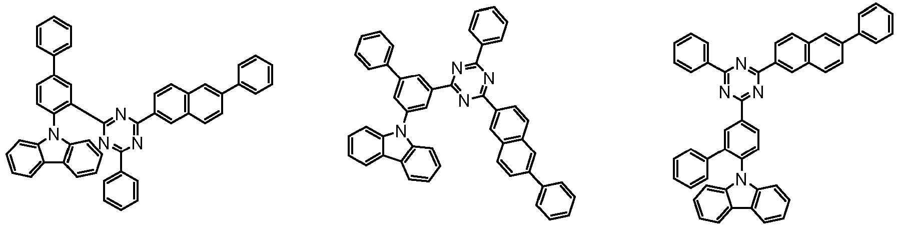

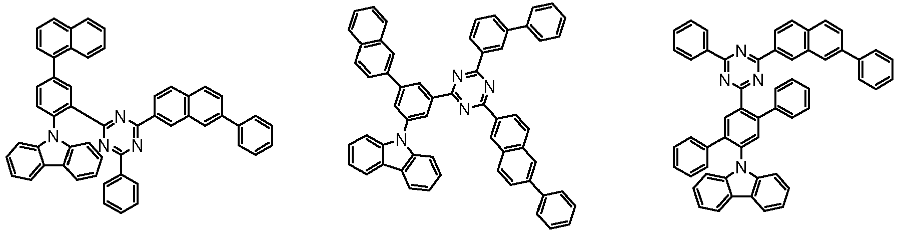

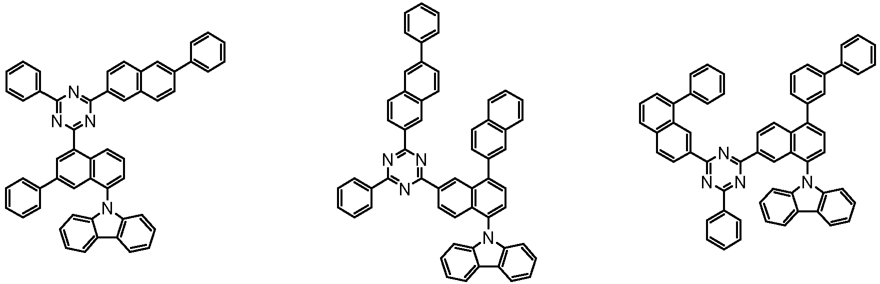

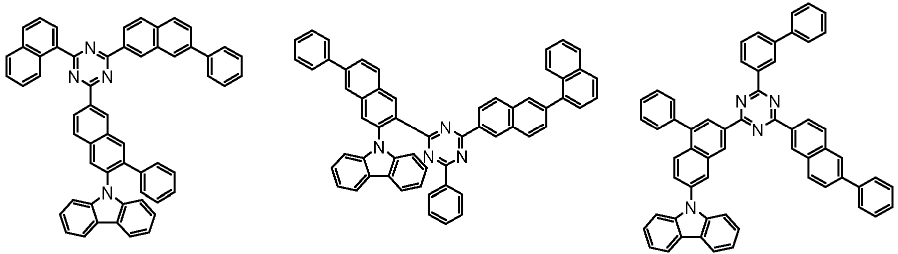

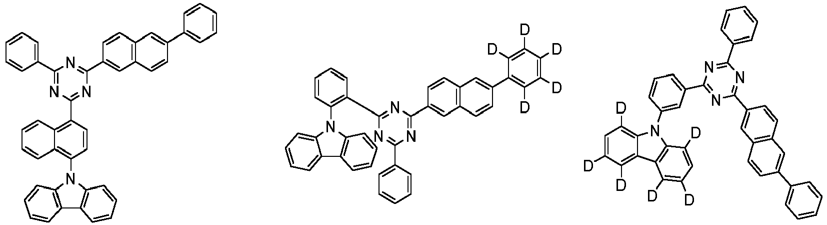

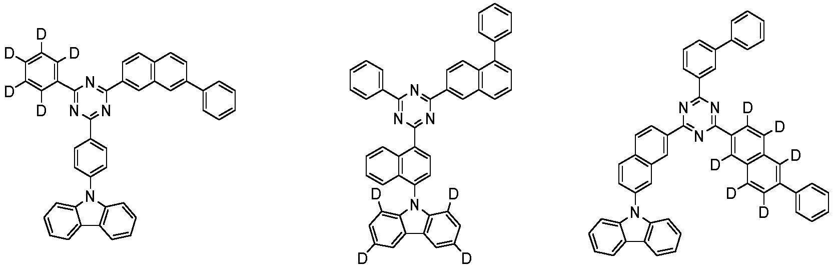

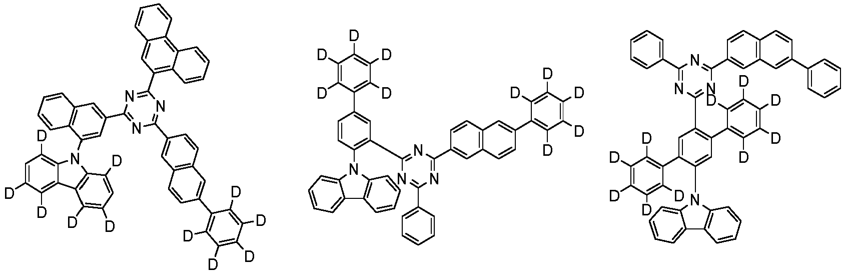

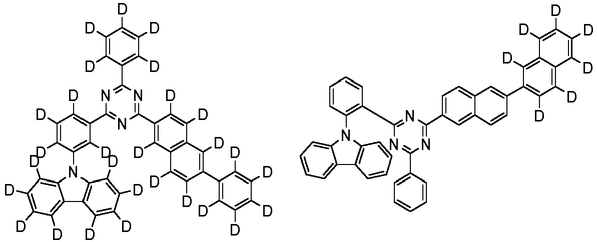

The compound represented by Chemical Formula 1 can be prepared by a preparation method as shown in the following Reaction Scheme 1 as an example, and other remaining compounds can also be prepared in a similar manner. 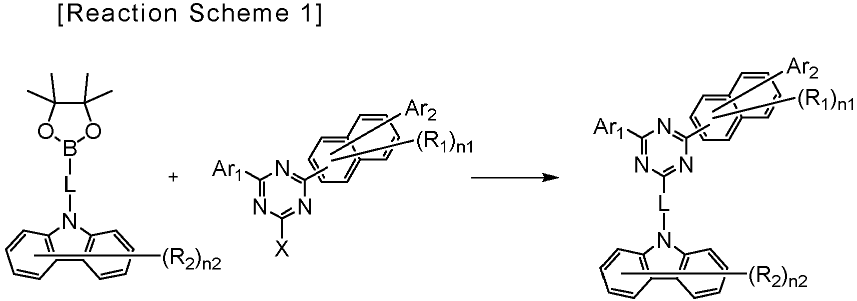 in Reaction Scheme 1, Ar₁, Ar₂, **L,** R₁, R₂, n1 and n2 are as defined in Chemical Formula 1, and X is halogen, preferably X is chloro or bromo.

Reaction Scheme 1 is a Suzuki coupling reaction, which is preferably carried out in the presence of a palladium catalyst and a base, and a reactive group for the Suzuki coupling reaction can be modified as known in the art. **The** preparation method can be further embodied in Preparation Examples described hereinafter.

Further, the present disclosure provides an organic light emitting device comprising a compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein one or more layers of the organic material layers includes the compound represented by Chemical Formula 1.

**The** organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, a light emitting layer, an electron transport layer, an electron injection layer and the like as the organic material layer. However, the structure of the organic light emitting device is not limited thereto, and it may include a smaller number of organic material layers.

Further, the organic material layer may include a light emitting layer, wherein the light emitting layer may include the compound represented by Chemical Formula 1.

Further, the organic material layer may include a hole transport layer, a hole injection layer, or a layer that simultaneously performs hole transport and hole injection, wherein the hole transport layer, the hole injection layer, or the layer that simultaneously performs hole transport and hole injection may include the compound represented by Chemical Formula 1.

Further, the organic material layer may include an electron transport layer, an electron injection layer, or an electron injection and transport layer, wherein the electron transport layer, the electron injection layer, or the electron injection and transport layer may include the compound represented by Chemical Formula 1.

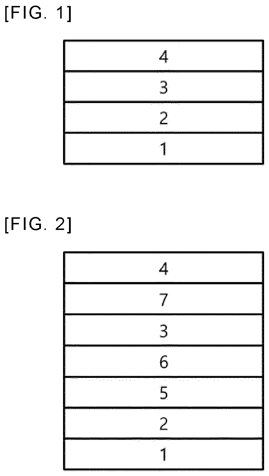

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of the organic light emitting device according to an embodiment of the present disclosure is illustrated in Figs. 1 and 2.

Fig. 1 shows an example of an organic light emitting device comprising a substrate 1, an anode 2, a light emitting layer 3, and a cathode 4. Fig. 2 shows an example of an organic light emitting device comprising a substrate 1, an anode 2, a hole injection layer 5, a hole transport layer 6, a light emitting layer 3, an electron injection and transport layer 7, and a cathode 4. In such a structure, the compound represented by Chemical Formula 1 may be included in the light emitting layer.

The organic light emitting device according to the present disclosure may be manufactured by materials and methods known in the art, except that at least one of the organic material layers includes the compound represented by Chemical Formula 1. Further, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device can be manufactured by sequentially depositing a cathode material, an organic material layer and an anode material on a substrate.

Further, the compound represented by Chemical Formula 1 can be formed into an organic material layer by a solution coating method as well as a vacuum deposition method at the time of manufacturing an organic light emitting device. Wherein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex (Alq₃); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, rubrene, and the like, but are not limited thereto.

The light emitting layer may include a host material and a dopant material. The host material includes a fused aromatic ring derivative, a heterocycle-containing compound, or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocycle-containing compounds include carbazole derivatives, dibenzofuran derivatives, ladder-type furan compounds, pyrimidine derivatives, and the like, but are not limited thereto. In particular, the compound according to the present disclosure can be used as a host for the light emitting layer.

Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The electron transport layer is a layer which receives electrons from an electron injection layer and transports the electrons to a light emitting layer, and an electron transport material is suitably a material which may receive electrons well from a cathode and transfer the electrons to a light emitting layer, and has a large mobility for electrons. Specific examples of the electron transport material include: an Al complex of 8-hydroxyquinoline; a complex including Alq₃; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

On the other hand, in the present disclosure, the "electron injection and transport layer" is a layer that performs both the roles of the electron injection layer and the electron transport layer, and the materials that perform the roles of each layer may be used alone or stacked and used in combination, without being limited thereto.

The organic light emitting device according to the present disclosure may be a bottom emission device, a top emission device, or a double-sided light emitting device, and particularly, may be a bottom emission device that requires relatively high luminous efficiency.

In addition, the compound represented by Chemical Formula 1 can be included in an organic solar cell or an organic transistor in addition to an organic light emitting device.

Below, the embodiments are described in more detail to assist in the understanding of the present disclosure. However, the following examples are provided for illustrative purposes only, and are not intended to limit the subject matter of the present disclosure.

### [PREPARATIONEXAMPLE]

### Preparation Example 1: Preparation of Compound 1

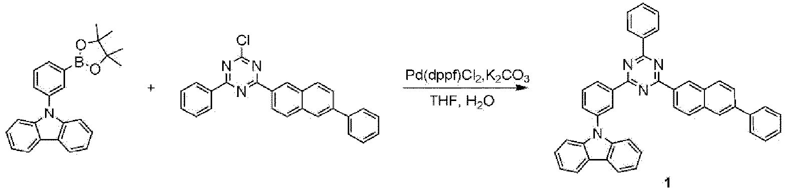

9-(3-(4,4,5,5-Tetramethyl-1,3,2-dioxaborolan-2-yl)phenyl)-9H-carbazole(15.0 g, 40.6 mmol) and 2-chloro-4-phenyl-6-(6-phenylnaphthalen-2-yl)-1,3,5-triazine(17.6 g, 44.7 mmol) were added to THF 300 ml under a nitrogen atmosphere, and the mixture was stirred and refluxed. Then, potassium carbonate(22.5 g, 162.5 mmol) was dissolved in 67 ml of water and added thereto, and the mixture was sufficiently stirred and then [1,1'-bis(diphenylphosphino)ferrocene]palladium(II) dichloride(0.9 g, 1.2 mmol) was added. After the reaction for 10 hours, the reaction mixture was cooled to room temperature, and then the organic layer and the aqueous layer were separated, and the organic layer was distilled. This was again dissolved in chloroform, washed twice with water, and then the organic layer was separated, anhydrous magnesium sulfate was added, stirred, then filtered, and the filtrate was distilled under reduced pressure. The concentrated compound was purified by a silica gel column chromatography to prepare 8.1g of Compound 1 (Yield: 33%, MS: [M+H]⁺= 602)

### Preparation Example 2: Preparation of Compound 2

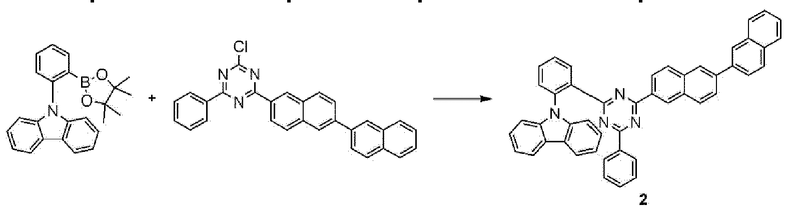

Compound 2 (MS[M+H]⁺= 652) was prepared in the same manner as in the Preparation of Compound 1, except that in Preparation Example 1, 9-(2-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole, and 2-([2,2'-binaphthalen]-6-yl)-4-chloro-6-phenyl-1,3,5-triazine was used instead of 2-chloro-4-phenyl-6-(6-phenylnaphthalen-2-yl)-1,3,5-triazine.

### Preparation Example 3: Preparation of Compound 3

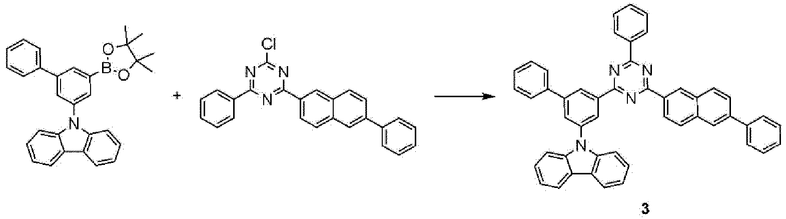

Compound 3 (MS[M+H]⁺= 678) was prepared in the same manner as in the Preparation of Compound 1, except that in Preparation Example 1, 9-(5-(4,4,5,5-tetramethyl-1,3,2-dioxaborolan-2-yl)-[1,1'-biphenyl]-3-yl)-9H-carbazole was used instead of 9-(3-(4,4,5,5-tetramethyl-1,3,2-dioxaborolan-2-yl)phenyl)-9H-carbazole.

### Preparation Example 4: Preparation of Compound 4

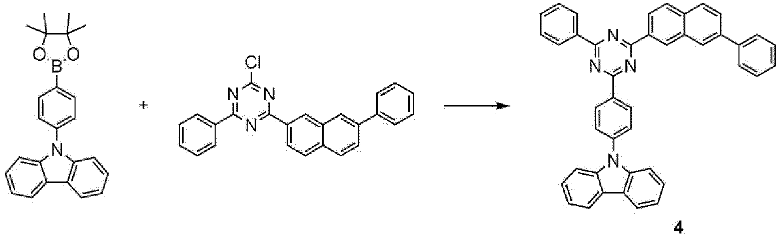

Compound 4 (MS[M+H]⁺= 602) was prepared in the same manner as in the Preparation of Compound 1, except that in Preparation Example 1, 9-(4-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole, and 2-chloro-4-phenyl-6-(7-phenylnaphthalen-2-yl)-1,3,5-triazine was used instead of 2-chloro-4-phenyl-6-(6-phenylnaphthalen-2-yl)-1,3,5-triazine.

### Preparation Example 5: Preparation of Compound 5

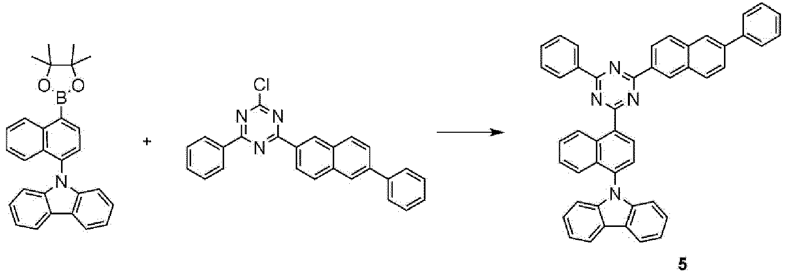

Compound 5 (MS[M+H]⁺= 652) was prepared in the same manner as in the Preparation of Compound 1, except that in Preparation Example 1, 9-(4-(4,4,5,5-tetramethyl-1,3,2-dioxaborolan-2-yl)naphthalen-1-yl)-9H-carbazole was used instead of 9-(3-(4,4,5,5-tetramethyl-1,3,2-dioxaborolan-2-yl)phenyl)-9H-carbazole.

### Preparation Example 6: Preparation of Compound 6

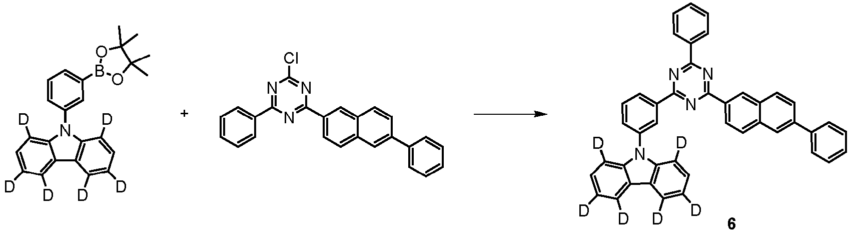

Compound 6 (MS[M+H]⁺= 608) was prepared in the same manner as in the Preparation of Compound 1, except that in Preparation Example 1, 9-(3-(4,4,5,5-tetramethyl-1,3,2-dioxaborolan-2-yl)phenyl)-9H-carbazole-1,3,4,5,6,8-d6 was used instead of 9-(3-(4,4,5,5-tetramethyl-1,3,2-dioxaborolan-2-yl)phenyl)-9H-carbazole.

### Preparation Example 7: Preparation of Compound 7

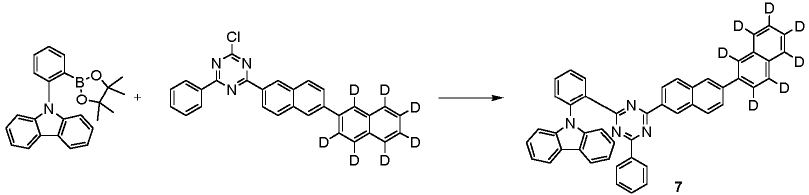

Compound 7 (MS[M+H]⁺= 659) was prepared in the same manner as in the Preparation of Compound 1, except that in Preparation Example 1, 9-(2-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole, and 2-([2,2'-binaphthalen]-6-yl-1',3',4',5',6',7',8'-d7)-4-chloro-6-phenyl-1,3,5-triazine was used instead of 2-chloro-4-phenyl-6-(6-phenylnaphthalen-2-yl)-1,3,5-triazine.

### [EXAMPLE]

### Comparative Example 1

A glass substrate on which a thin film of ITO (indium tin oxide) was coated in a thickness of 1,400 Å was put into distilled water containing the detergent dissolved therein and washed by the ultrasonic wave. In this case, the used detergent was Decon^{™} CON705 product commercially available from Fischer Co. and the distilled water was one which had been twice filtered by using a 0.22 µm sterilizing filter commercially available from Millipore Co. The ITO was washed for 30 minutes, and ultrasonic washing was then repeated twice for 10 minutes by using distilled water. After the washing with distilled water was completed, the substrate was ultrasonically washed with the solvents of isopropyl alcohol, acetone and methanol for 10 minutes, and dried, after which it was transported to a plasma cleaner. Then, the substrate was cleaned with oxygen plasma for 5 minutes, and then transferred to a vacuum evaporator.

On the ITO transparent electrode thus prepared, the following Compound HI-A and the following Compound LG-101 were sequentially thermally vacuum-deposited to a thickness of 800Å and 50Å, respectively, to form a hole injection layer. The following Compound HT-A was vacuum-deposited thereon to a thickness of 800 Å as a hole transport layer, and then, the following Compound EB-A was thermally vacuum-deposited to a thickness of 600 Å as an electron blocking layer. Compound RH-A was applied as a host for the light emitting layer, and Compound RD-A was applied as a dopant, and the host and dopant were vacuum-deposited at a weight ratio of 98:2 to a thickness of 400 Å. Then, the following Compound ET-A and Compound Liq were thermally vacuum-deposited at a ratio of 1:1 to a thickness of 360 Å, and then Liq was vacuum-deposited to a thickness of 5 Å as an electron injection and transport layer.

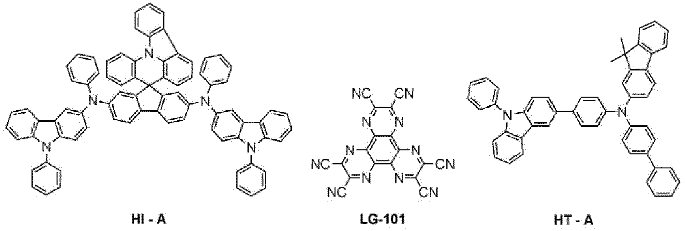

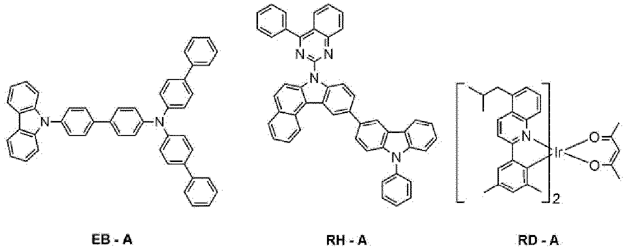

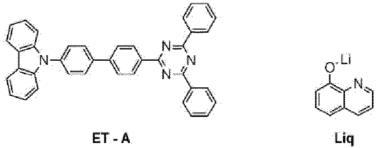

Magnesium and silver were sequentially deposited in a weight ratio of 10: 1 to a thickness of 220 Å, and aluminum to a thickness of 1000 Å on the electron injection and transport layer to form a cathode, thereby completing the manufacture of an organic light emitting device.

In the above-mentioned processes, the deposition rates of the organic materials were maintained at 0.4 ~0.7 Å/sec, the deposition rates of magnesium and silver were maintained at 2 Å/sec, and the degree of vacuum during the deposition was maintained at 2*10⁻⁷ ~ 5*10⁻⁶ torr, thereby manufacturing an organic light emitting device.

### Examples 1 to 7 and Comparative Examples 2 to 5

The organic light emitting devices of Examples 1 to 7 and Comparative Examples 2 to 5 were respectively manufactured in the same manner as in Comparative Example 1, except that in Comparative Example 1, the compounds shown in Table 1 below were used instead of RH-A.

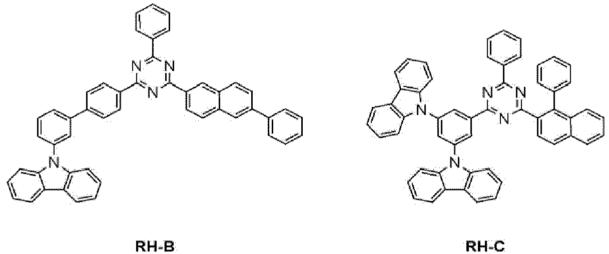

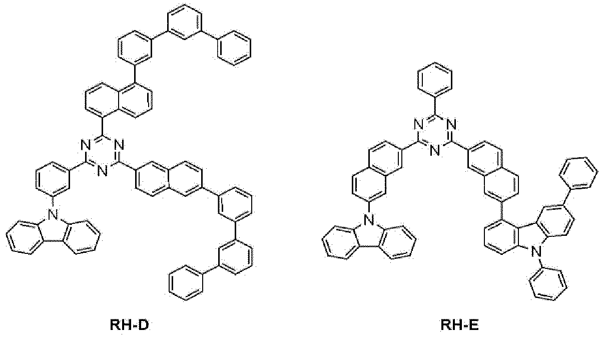

### [Experimental Example]

The voltage, efficiency and lifetime were measured by applying a current to the organic light emitting devices manufactured in Examples 1 to 7 and Comparative Examples 1 to 5, and the results are shown in Table 1 below. At this time, the voltage and efficiency were measured by applying a current density of 10 mA/cm², and LT97 (lifetime) means the time required for the luminance to be reduced to 97% of the initial luminance at a current density of 20 mA/cm².

**[Table 1]**

| | Host for light emitting layer | @ 10mA/cm² | | @ 20mA/cm² |
|---|---|---|---|---|
| | | V | cd/A | LT97 (hr) |
| Example 1 | Compound 1 | 4.73 | 17.8 | 82 |
| Example 2 | Compound 2 | 4.80 | 18.2 | 77 |
| Example 3 | Compound 3 | 4.84 | 17.9 | 93 |
| Example 4 | Compound 4 | 4.76 | 18.9 | 96 |
| Example 5 | Compound 5 | 4.79 | 18.4 | 74 |
| Example 6 | Compound 6 | 4.73 | 17.7 | 109 |
| Example 7 | Compound 7 | 4.80 | 18.1 | 106 |
| Comparativ e Example 1 | Compound RH-A | 5.43 | 14.2 | 42 |
| Comparativ e Example 2 | Compound RH-B | 5.51 | 11.4 | 52 |
| Comparativ e Example 3 | Compound RH-C | 5.98 | 9.6 | 38 |
| Comparativ e Example 4 | Compound RH-D | 6.23 | 5.2 | 14 |
| Comparativ e Example 5 | Compound RH-E | 6.19 | 6.3 | 18 |

From the results of Table 1 above, it was confirmed that when the compounds having the structure of Chemical Formula 1 were applied to the light emitting layer of an organic light emitting device, a device having low voltage, high efficiency, and long lifetime characteristics can be obtained.

### [Description of symbols]

| | | | |
|---|---|---|---|
| 1: | substrate | 2: | anode |
| 3: | light emitting layer | 4: | cathode |
| 5: | hole injection layer | 6: | hole transport layer |
| 7: | electron injection and transport layer | | |

## Claims

1. A compound represented by the following Chemical Formula 1 : 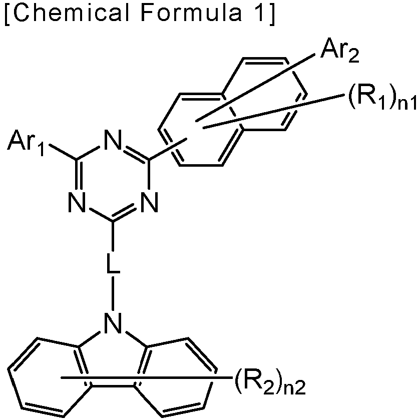 in Chemical Formula 1,
Ar₁ is selected from the group consisting of: 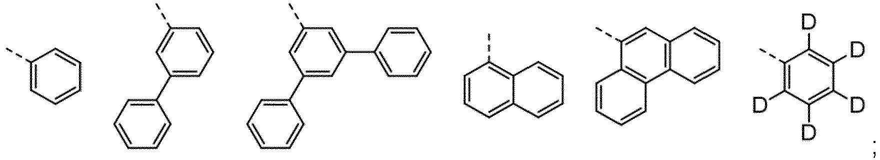
Ar₂ is phenyl or naphthyl, wherein the Ar₂ is unsubstituted or substituted with at least one deuterium;
L is selected from the group consisting of: 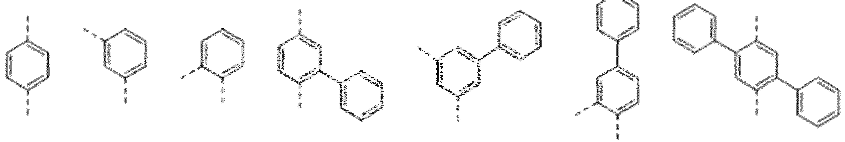 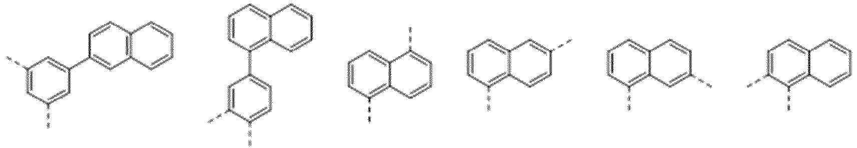 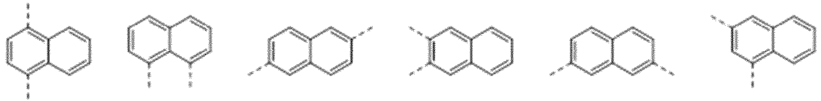 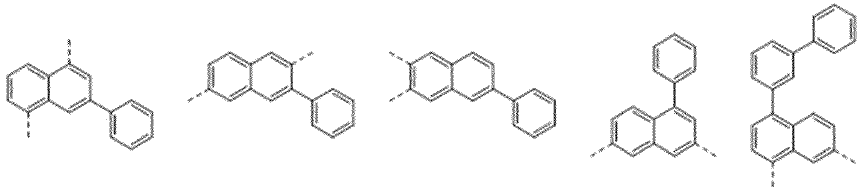 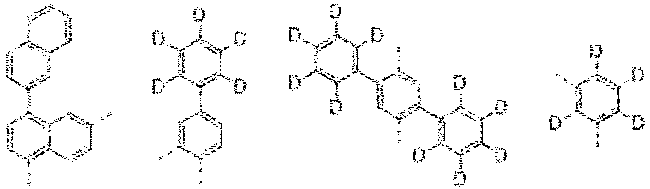
R₁ and R₂ are each independently hydrogen or deuterium,
n1 is an integer of 0 to 6, and
n2 is an integer of 0 to 8.

2. The compound according to claim 1 wherein:
the Chemical Formula 1 is represented by any one of the following Chemical Formulas 1-1 to 1-6: 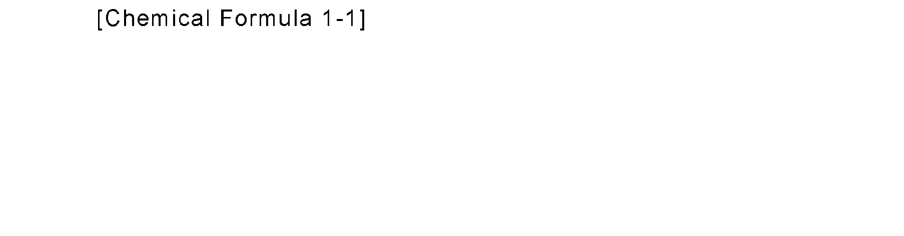 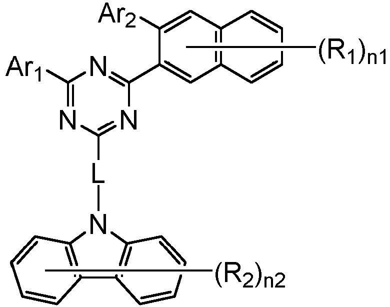 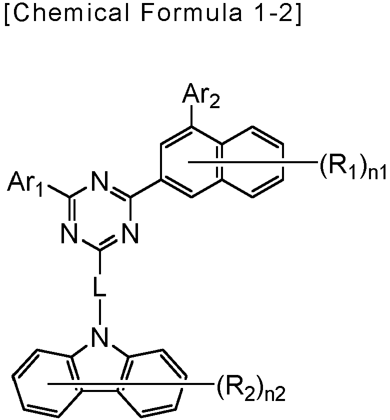 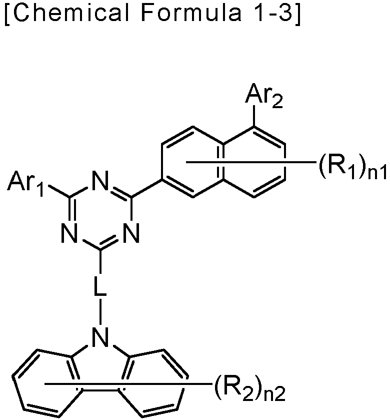 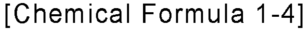 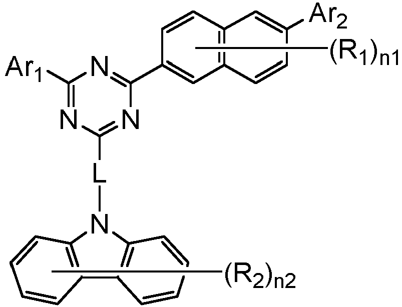 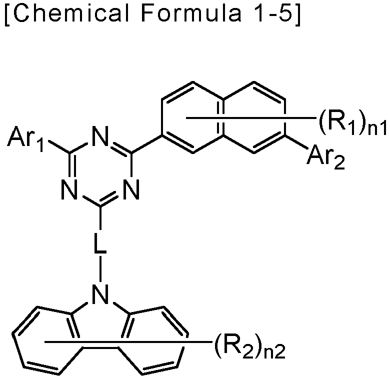 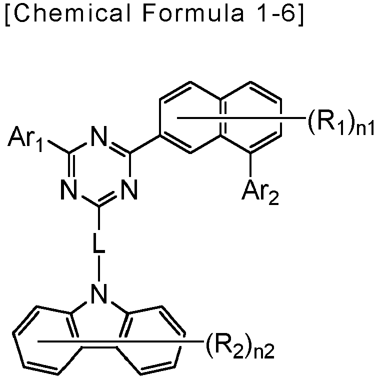
in Chemical Formulas 1-1 to 1-6,
Ar₁, Ar₂, L, R₁, R₂, n1 and n2 are as defined in claim 1.

3. The compound according to claim 1, wherein Ar₂ is selected from the group consisting of:

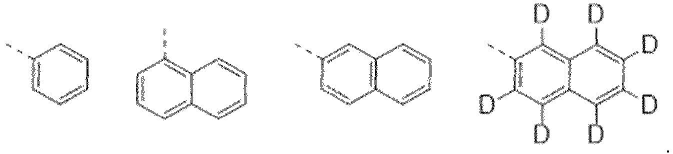

4. The compound according to claim 1 wherein:
the compound represented by Chemical Formula 1 is any one selected from the group consisting of:

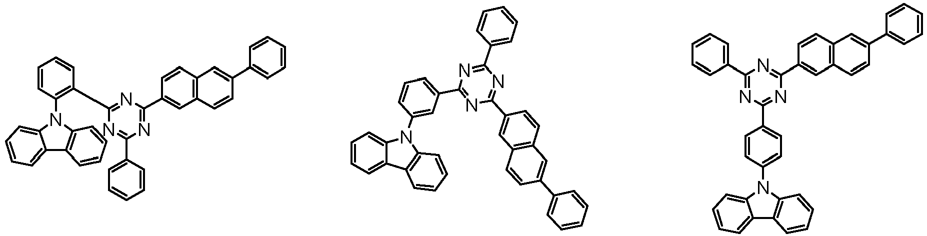

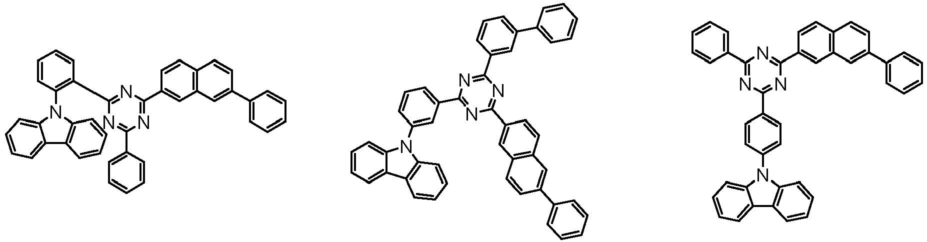

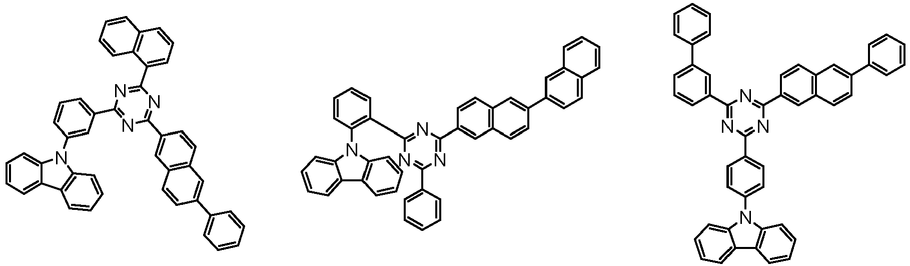

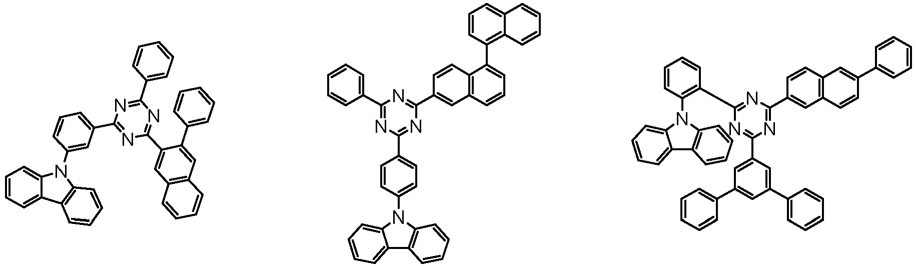

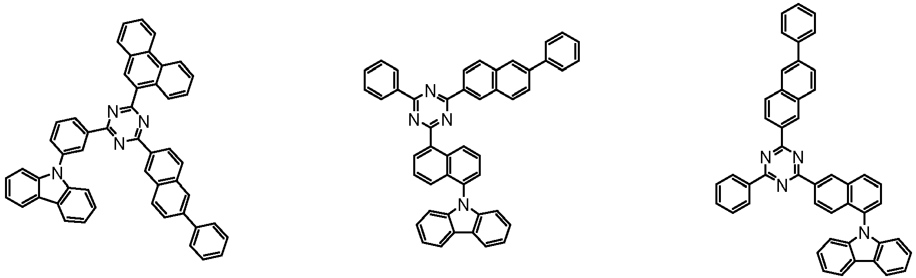

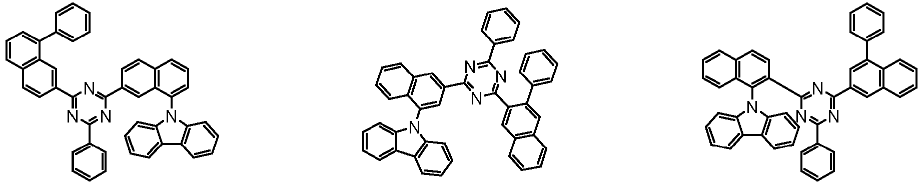

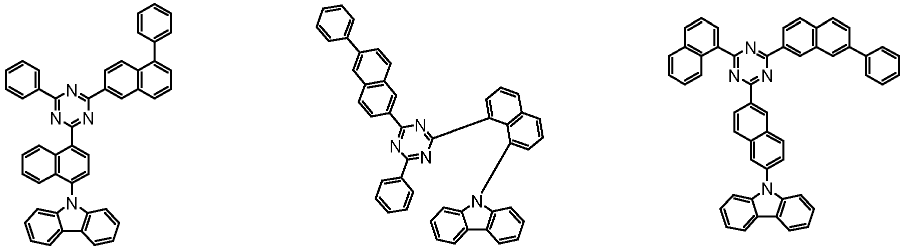

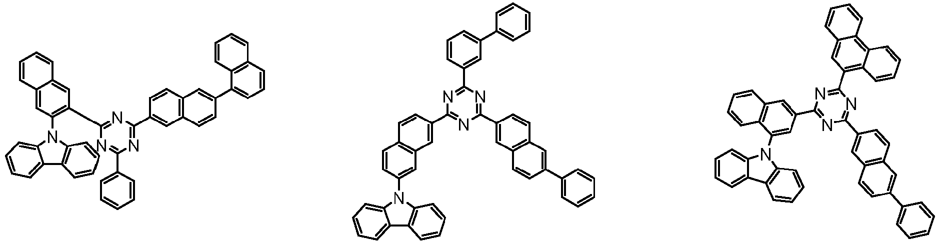

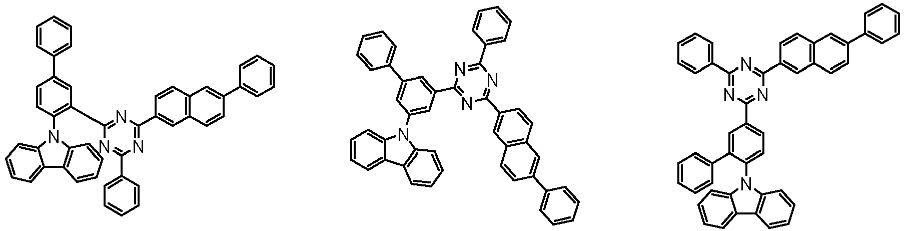

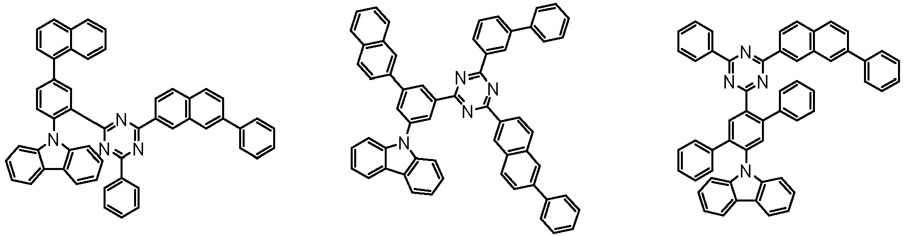

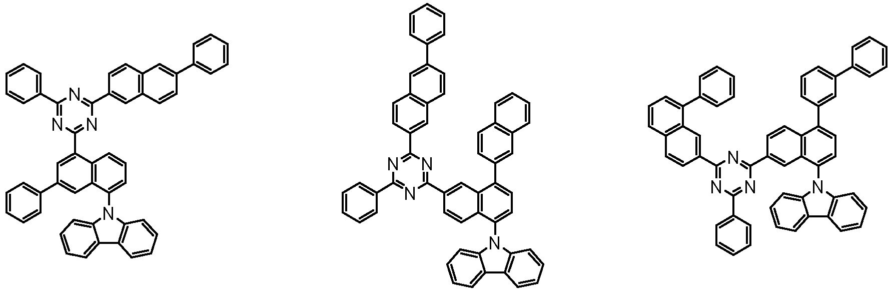

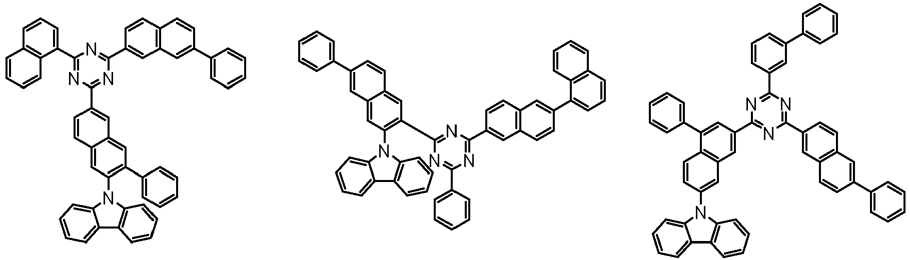

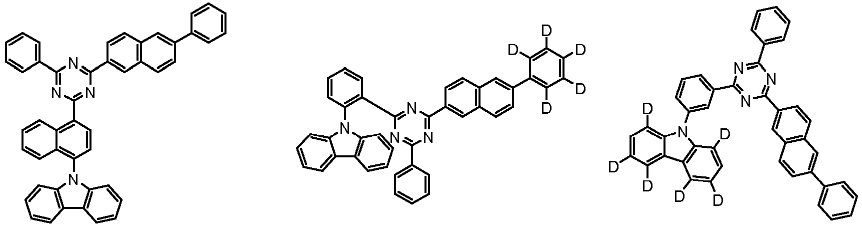

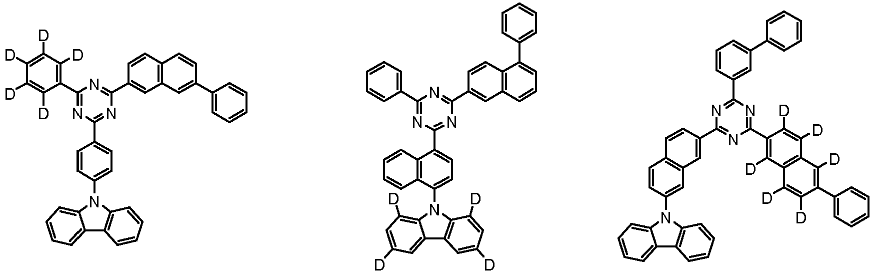

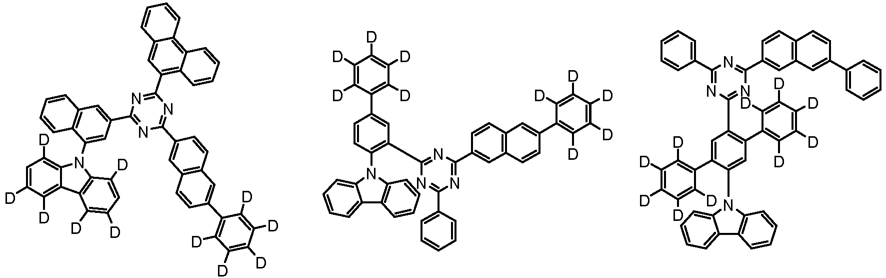

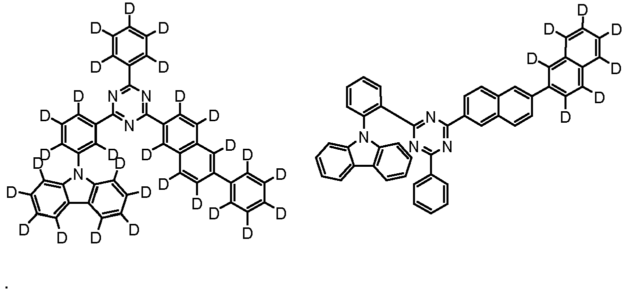

5. An organic light emitting device comprising: a first electrode; a second electrode that is provided opposite to the first electrode; and one or more organic material layers that are provided between the first electrode and the second electrode, wherein one or more layers of the organic material layers comprises the compound of any one of claims 1 to 4.

6. The organic light emitting device according to claim 5, wherein:
the organic material layer is a light emitting layer.

## Patentansprüche

1. Durch die folgende chemische Formel 1 dargestellte Verbindung: 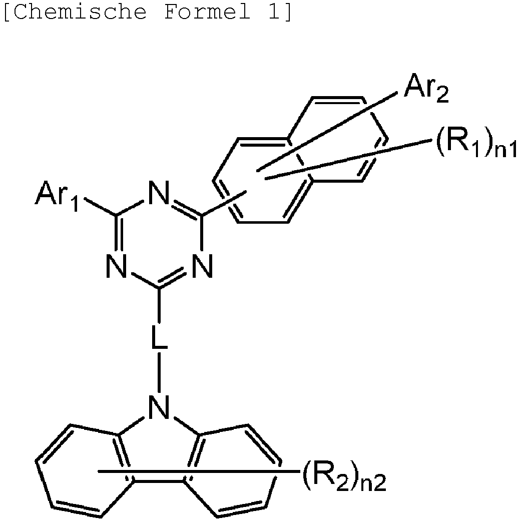 worin in chemischer Formel 1
Ar₁ ausgewählt ist aus der Gruppe, bestehend aus: 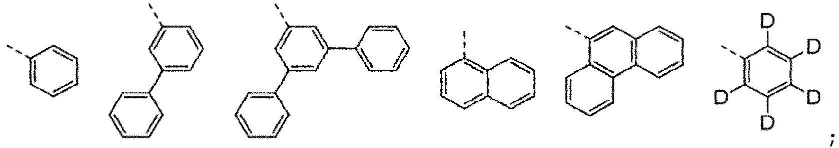
Ar₂ Phenyl oder Naphthyl ist, worin Ar₂ unsubstituiert ist oder substituiert ist mit zumindest einem Deuterium;
L ausgewählt ist aus der Gruppe, bestehend aus: 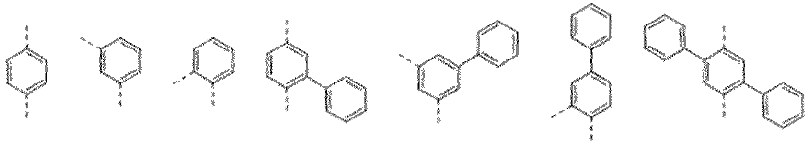 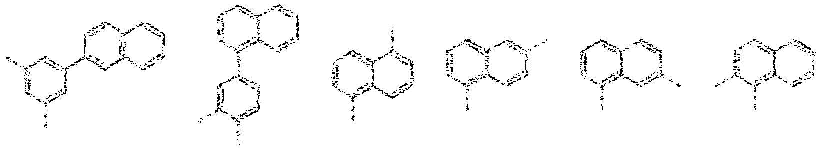 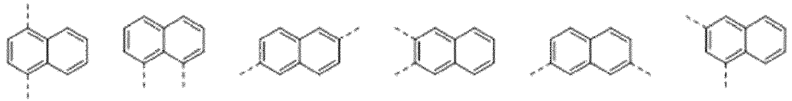 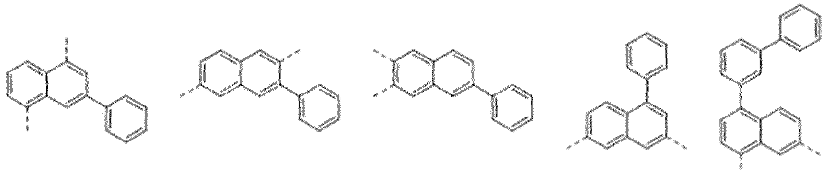 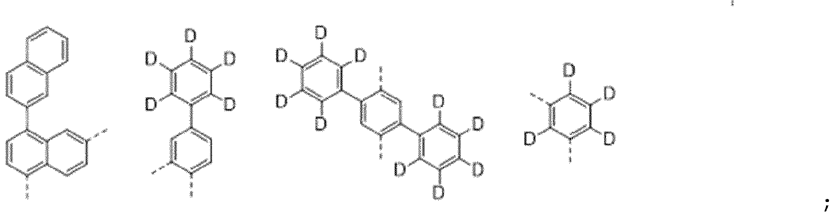
R₁ und R₂ jeweils unabhängig Wasserstoff oder Deuterium sind,
n1 eine ganze Zahl von 0 bis 6 ist und
n2 eine ganze Zahl von 0 bis 8 ist.

2. Verbindung gemäß Anspruch 1, worin:
die chemische Formel 1 dargestellt ist durch irgendeine der chemischen Formeln 1-1 bis 1-6: 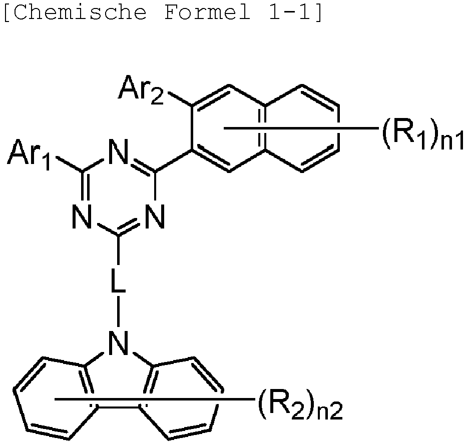 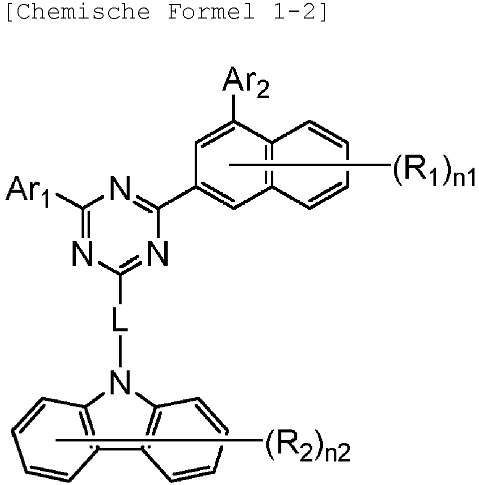 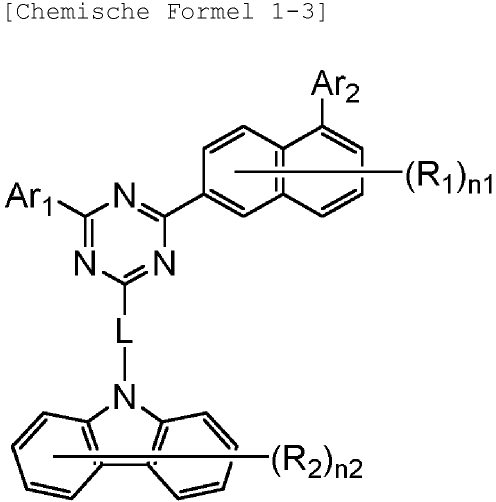 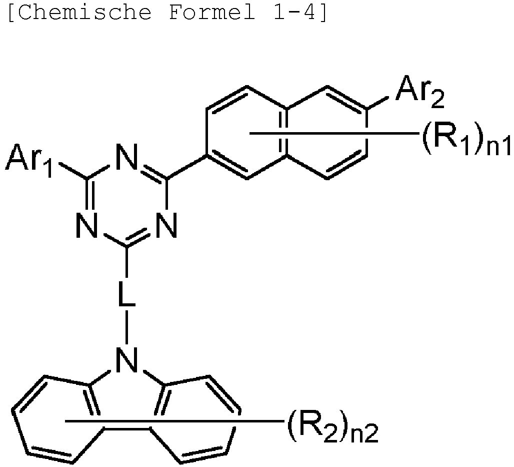 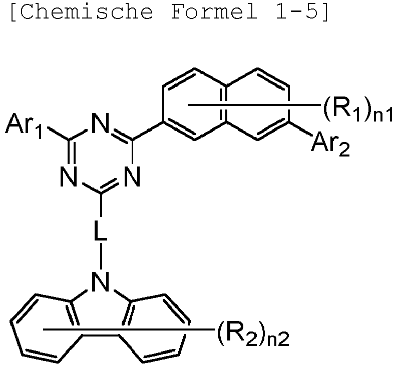 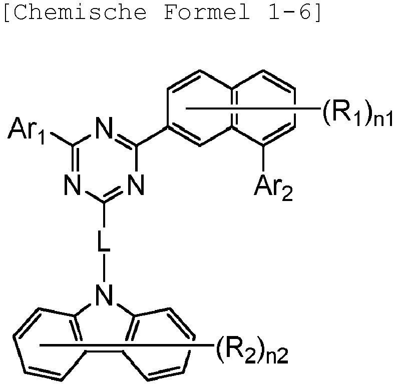
worin in den chemischen Formeln 1-1 bis 1-6
Ar₁, Ar₂, L, R₁, R₂, n1 und n2 wie in Anspruch 1 definiert sind.

3. Verbindung gemäß Anspruch 1, worin Ar₂ ausgewählt ist aus der Gruppe, bestehend aus:

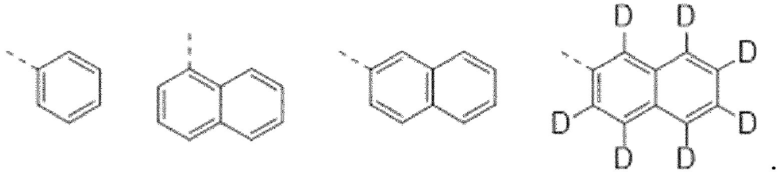

4. Verbindung gemäß Anspruch 1, worin:
die durch die chemische Formel 1 dargestellte Verbindung irgendeine ist, die ausgewählt ist aus der Gruppe, bestehend aus:

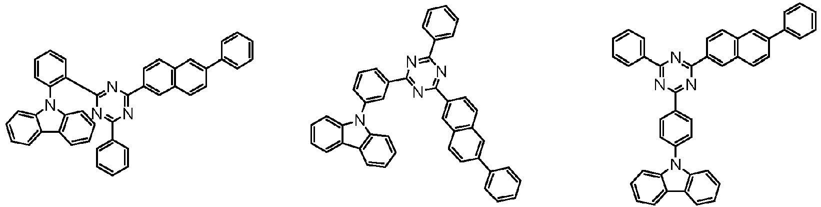

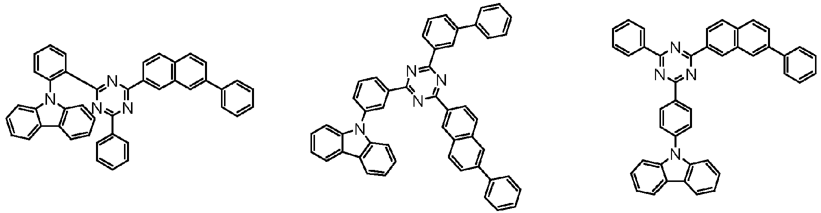

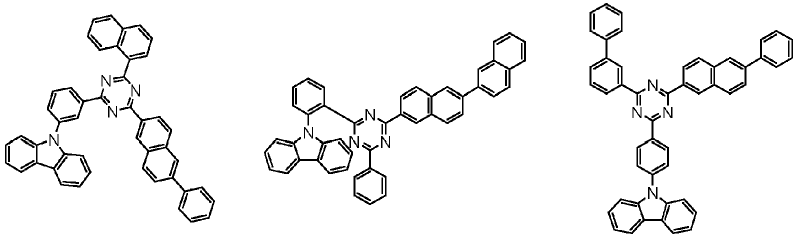

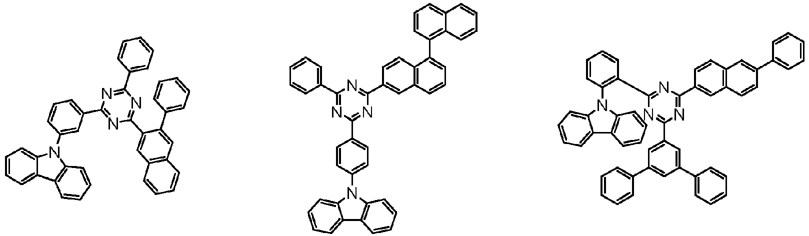

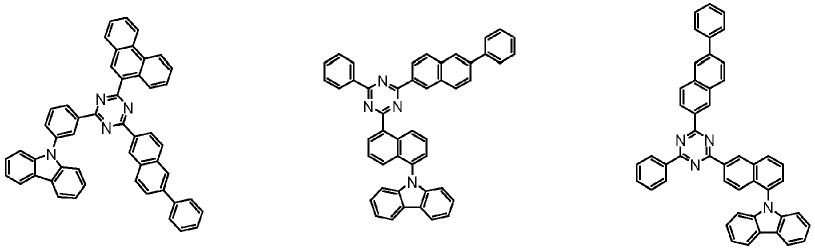

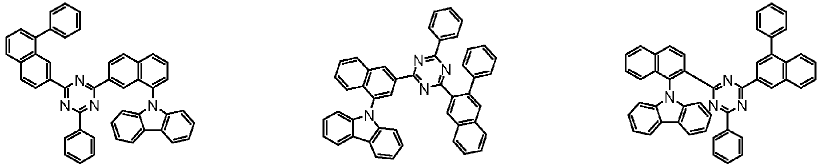

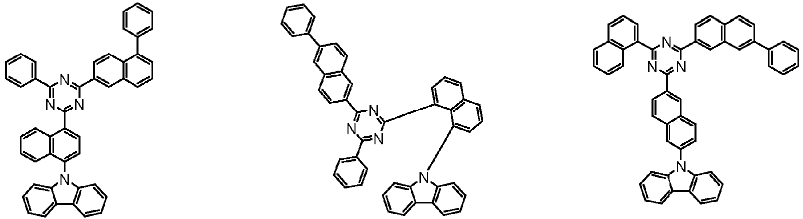

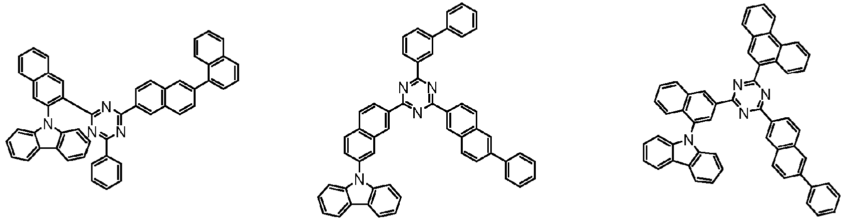

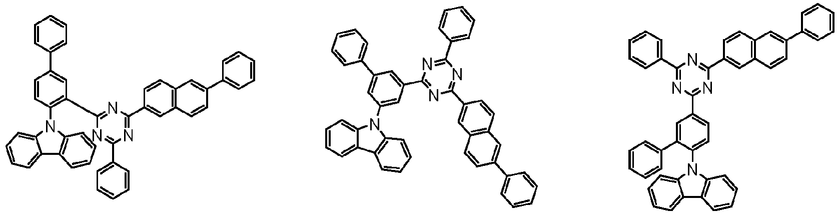

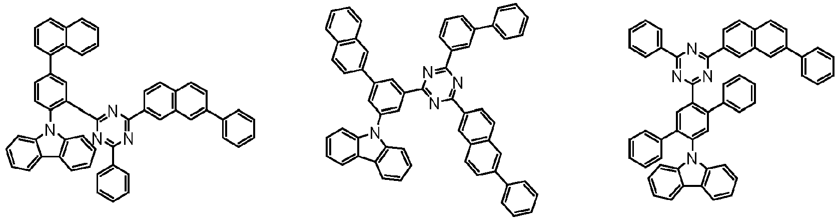

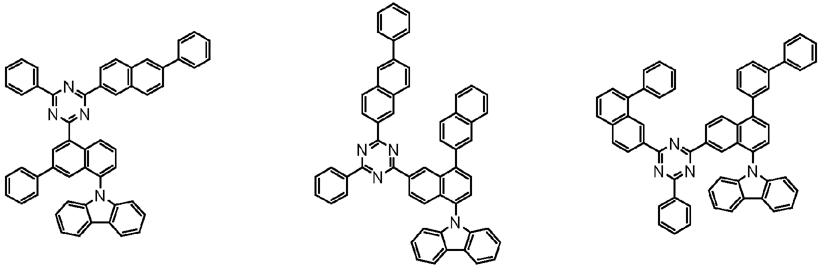

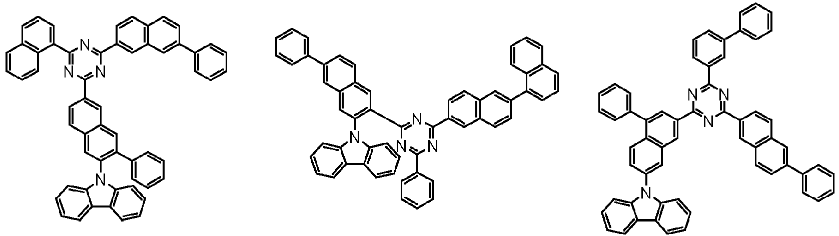

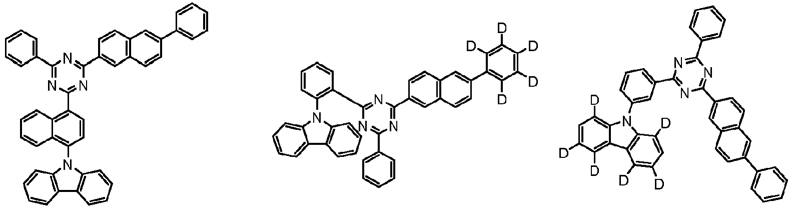

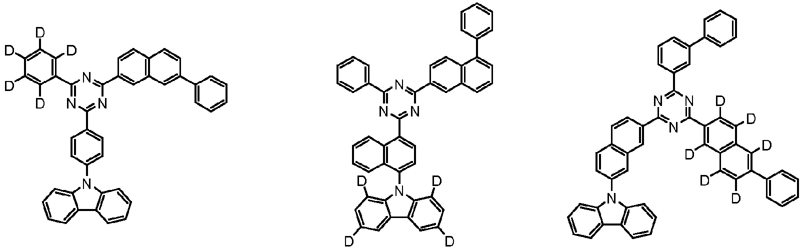

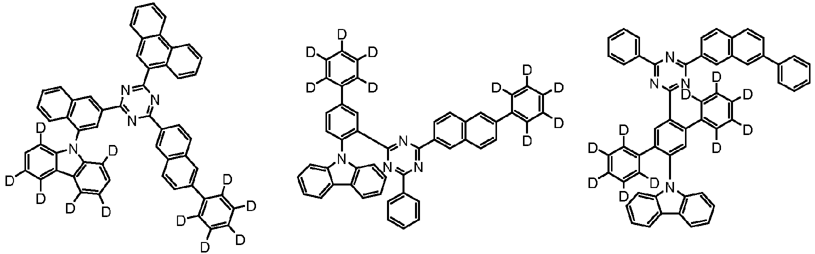

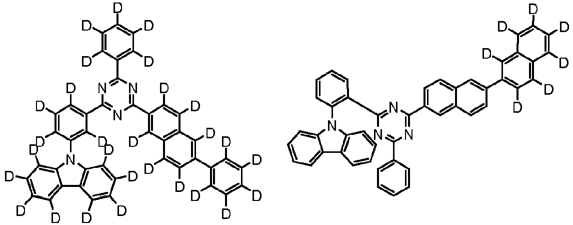

5. Organische lichtemittierende Vorrichtung, umfassend:
eine erste Elektrode, eine zweite Elektrode, die der ersten Elektrode gegenüberliegend vorgesehen ist; und
eine oder mehr organische Materialschichten, die zwischen der ersten Elektrode und der zweiten Elektrode vorgesehen ist/sind, worin eine oder mehr Schichten der organischen Materialschicht(en) die Verbindung gemäß irgendeinem der Ansprüche 1 bis 4 umfasst.

6. Organische lichtemittierende Vorrichtung gemäß Anspruch 5, worin:
die organische Materialschicht eine lichtemittierende Schicht ist.

## Revendications

1. Composé représenté par la Formule chimique 1 suivante : 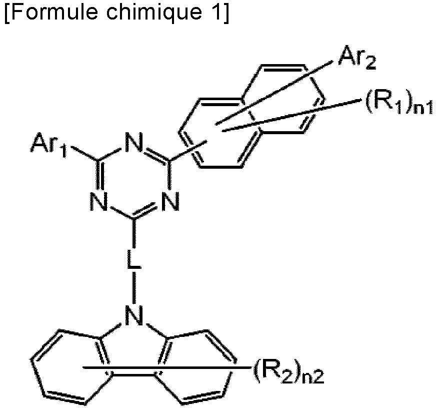 dans la Formule chimique 1,
Ar₁ est sélectionné parmi le groupe constitué de : 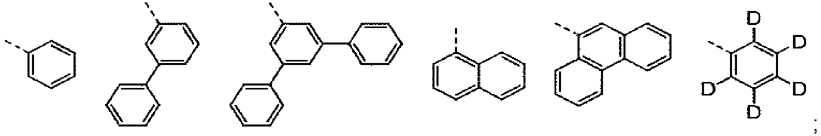
Ar₂ est phényle ou naphthyle, dans lequel l'Ar₂ est non substitué ou substitué par au moins un deutérium ;
L est sélectionné parmi le groupe constitué de : 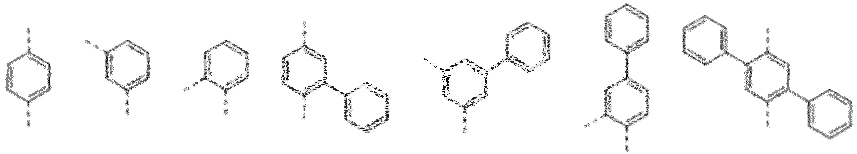 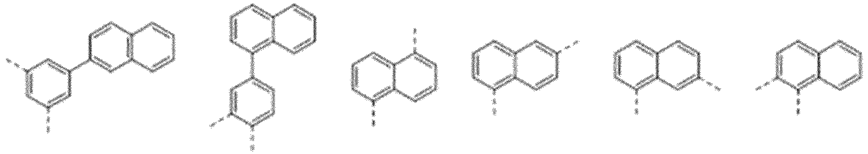 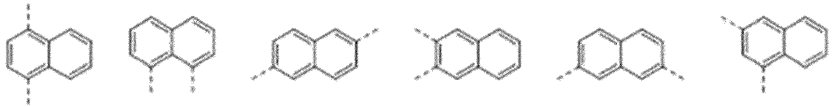 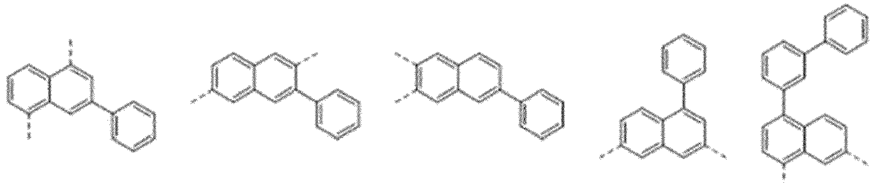 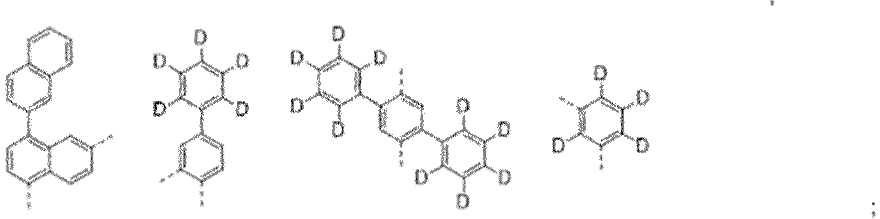
R₁ et R₂ sont chacun indépendamment hydrogène ou deutérium ;
n1 est un nombre entier de 0 à 6, et
n2 est un nombre entier de 0 à 8.

2. Composé selon la revendication 1 dans lequel :
la Formule chimique 1 est représentée par l'une quelconque des formules chimiques 1-1 à 1-6 suivantes : 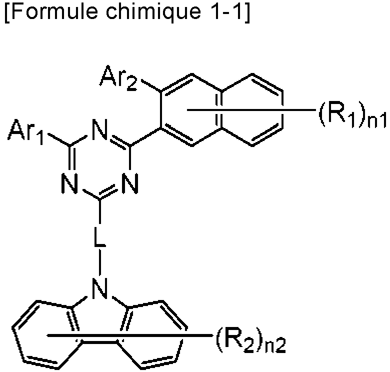 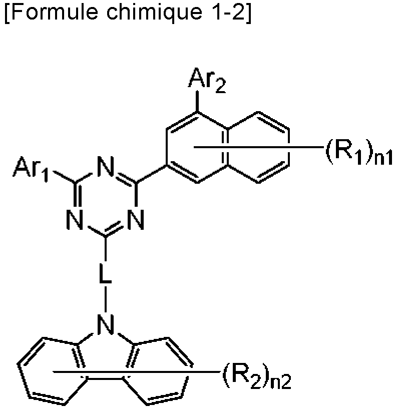 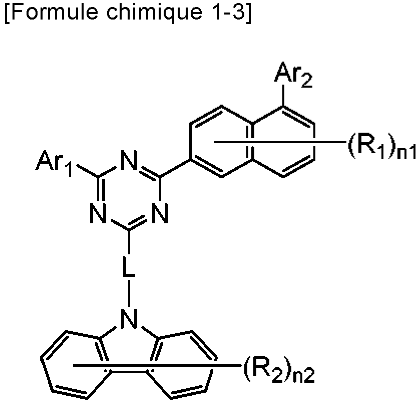 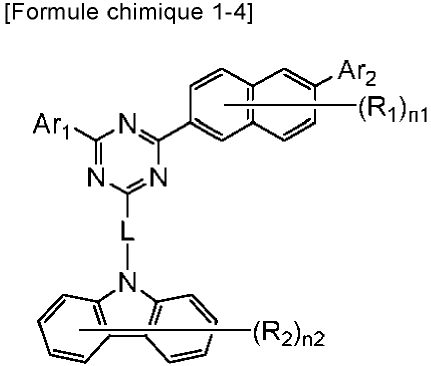 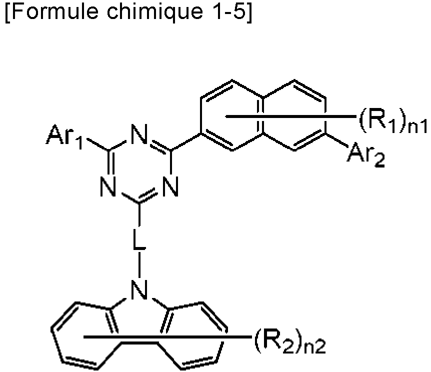 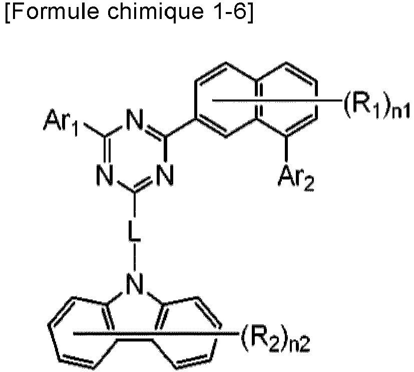
dans les Formules chimiques 1-1 à 1-6,
Ar₁, Ar₂, L, R₁, R₂, n1 et n2 sont tels que définis dans la revendication 1.

3. Composé selon la revendication 1, dans lequel Ar₂ est sélectionné parmi le groupe constitué de :

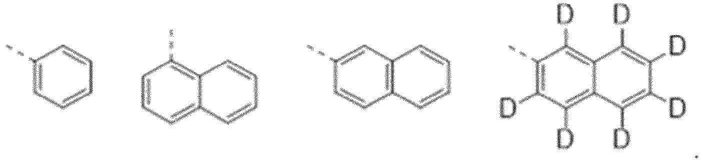

4. Composé selon la revendication 1, dans lequel :
le composé représenté par la Formule chimique 1 est l'un quelconque parmi le groupe constitué de :

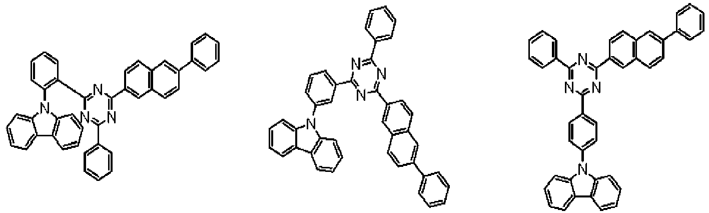

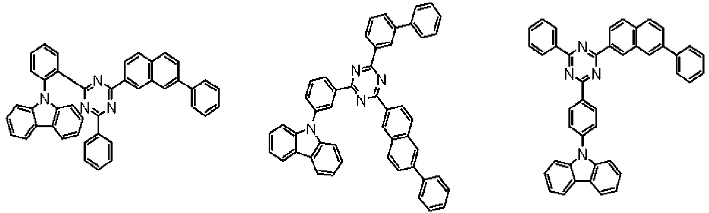

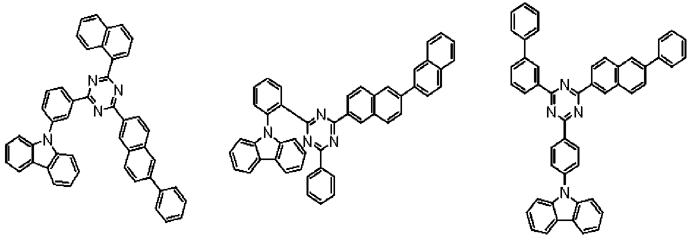

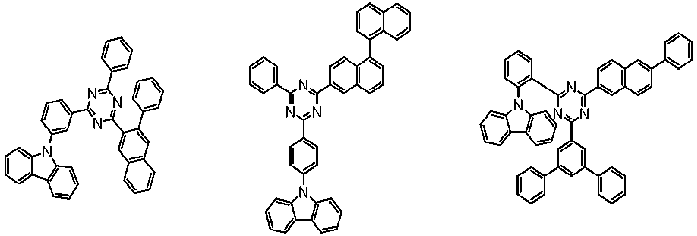

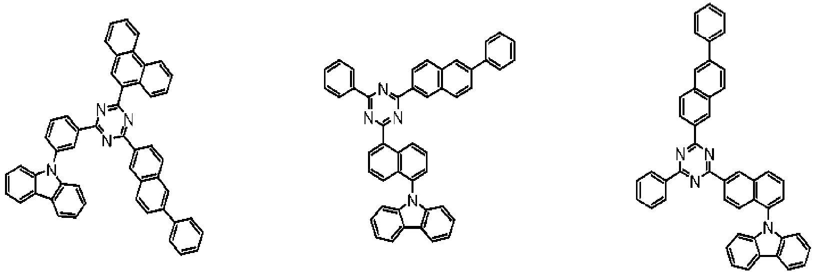

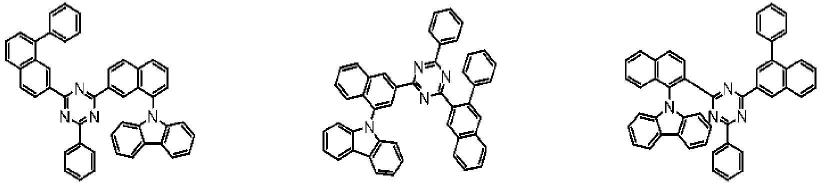

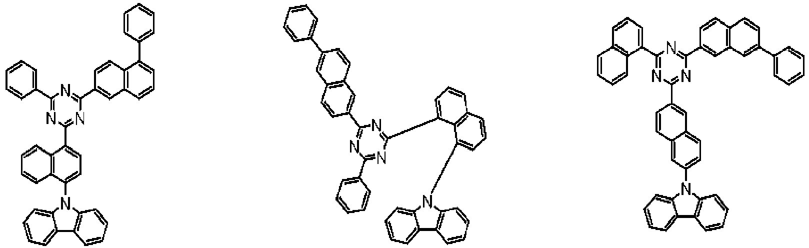

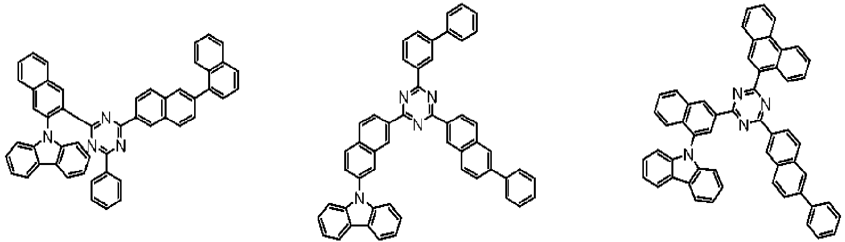

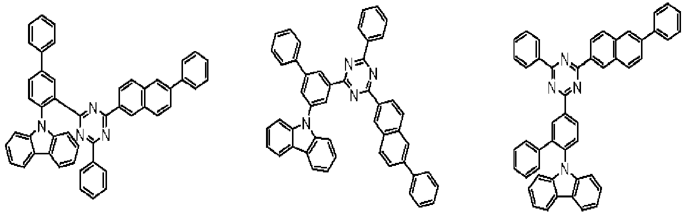

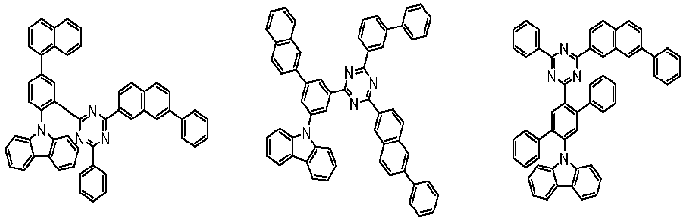

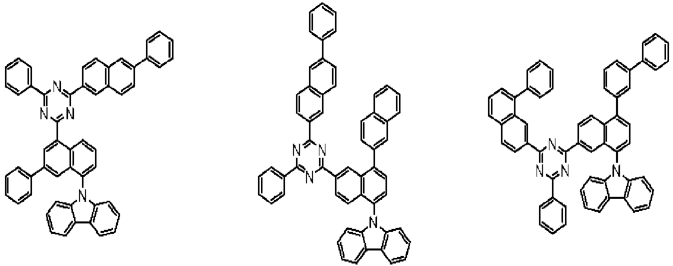

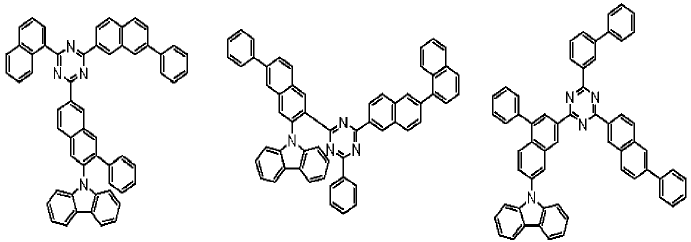

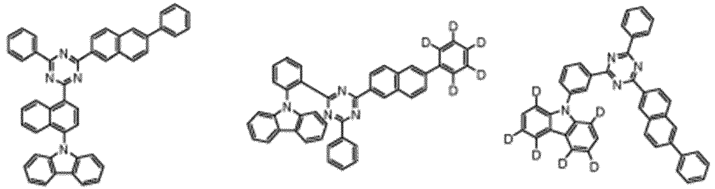

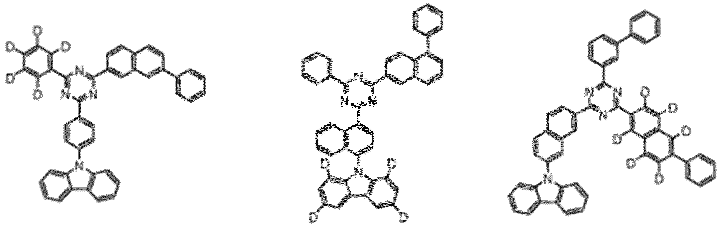

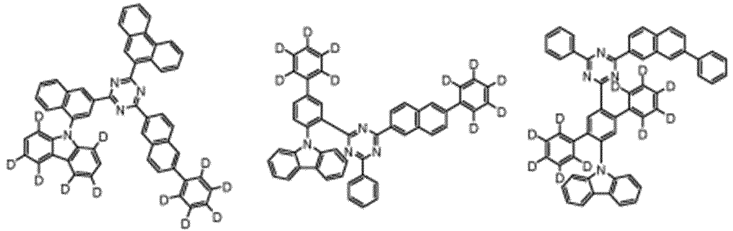

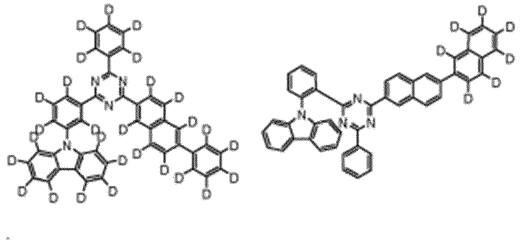

5. Dispositif électroluminescent organique comprenant : une première électrode ; une seconde électrode qui est fournie à l'opposé de la première électrode ; et une ou plusieurs couches de matériau organique qui sont fournies entre la première électrode et la seconde électrode, dans lequel une ou plusieurs couches des couches de matériau organique comprennent le composé selon l'une quelconque des revendications 1 à 4.

6. Dispositif électroluminescent organique selon la revendication 5, dans lequel : la couche de matériau organique est une couche électroluminescente.
